# EUROPEAN PATENT APPLICATION

(11) **EP 0 641 550 A1**
(43) Date of publication of application: **08.03.1995**
(21) Application number: 90312519.3
(22) Date of filing: 16.11.1990
(51) Int. Cl.: A61F 5/01

(54) **Orthotic device**

(71) Applicant: SECRETARY OF STATE FOR HEALTH IN HER BRITANNIC MAJESTY'S GOV. OF THE UNITED KINGDOM OF GREAT BRITAIN AND NORTHERN IRELAND, London WC1B 5EP (GB)
(72) Inventor: Farmer, Ian Richard, Nr Oswestry, Shropshire (GB); Hodnett, Christine, Cockshutt, Nr. Ellesmere, Shropshire (GB); Jones, Norman John, St Martins, Nr Oswestry, Shropshire (GB); Major, Richard Edward, Oswestry, Shropshire (GB); Moore, Michelle, Rhydycroesau, Oswestry, Shropshire (GB); Poiner, Raymond, Oswestry, Shropshire (GB); Roberts, David, Nr Oswestry, Shropshire (GB); Stallard, John, Oswestry, Shropshire (GB)
(74) Representative: Skelton, Stephen Richard

(57) **Abstract**

An orthotic device suitable for the investigation and treatment of joint malfunctions includes a first member (10) rotatably connected to a second member (14). Two adjustable limits (41. 42) restrict the relative rotation of the members, (10, 14) and over- ride means (71, 72, 73) are provided to allow rotation beyond the limits (41, 42). Means (73, 50, 51) are also provided for locking the first (10) and second (14) members in at least one fixed position

## Description

The present invention relates to orthotic devices for the treatment of patients with walking disabilities. The device is suitable for use by patients with a variety of problems, but is particularly concerned with the treatment of victims of cerebral palsy.

Cerebral palsy victims suffer from motor control problems which vary considerably from patient to patient. Consequently surgeons have great difficulties in deciding on the best type of walking aid for any particular patient. The best aid can often be decided only by trial and error. This involves the patient's trying in sequence a plurality of aids each allowing a different range of movement. This is not only time consuming for the surgeon and his assistants but also results in tiredness of the patient such that several sessions may be necessary for a successful diagnosis of appropriate equipment be made. Furthermore the condition of a patient can be expected to change with time. with a result that changes in equipment must be made. With presently available equipment this requires a repeat of the above described test procedure.

Orthotic devices are known with articulating joints where the degree of articulation in either direction can be adjusted by stops and in which at least one of the stops can be overridden to. for example, allow a standing patient to adopt a sitting position. An example is UK-A-2168106. The device described in this Application, however, has no locking position, and for cerebral palsy victims in particular it is desirable that an orthotic walking aid should be lockable in a standing position, a sitting position or, preferably, both standing and sitting positions.

There is, therefore, a need for an orthotic device which enables adjustment of the various movement parameters to be made whilst fitted to a patient, which can be set to a preferred adjustment when this has been optimised, and which can be adjusted in situ to allow for changes in a patient's condition.

According to the present invention an orthotic device for the investigation and treatment of joint malfunctions includes a first member rotatably connected to a second member, means for allowing relative rotation of the first and second members over a range between two adjustable limits and over-ride means for allowing relative rotation of the first and second members beyond these limits, and locking means for locking the first and second members in at least one fixed position

A particular device according to the invention is used for a hip joint. There will be usually two such devices, one for each hip, on each patient, and these will usually be mounted on a common body support. Preferably each device will be so constructed that a cross-linking arrangement can be fitted thereto without removal from a patient. Cross-linking devices, whereby two limbs are constrained to move at the same time and usually consisting of two bowden cable arrangements, are well-known in the art.

In a preferred form of device the first and second members are arranged to rotate about a pivot, the first member having two stop members whose positions are adjustable along a segment of a circle at a fixed common radius from the pivot. The second member carries a plunger device having means for contacting the stop members on the first device and so adapted that it can be moved to a position where the stop members can be overriden. A preferred form of plunger device is of cylindrical form having at one end a tongue member which can be adapted to contact the stop member, the tongue member lying along a chord offset from the diameter of the plunger such that rotation of the plunger allows it to be moved into or out of positions where it contacts the atop members as required. The first member preferably contains means whereby, when the tongue on the plunger is in a position where it does not contact the stop members it can be used to lock the first and second members in at least one fixed position.

The plunger member is perferably connected to the second member in such a way that it can be removed to allow part of a cross-linking device to be attached to the second member.

One embodiment of the invention will now be described, by way of example only, with reference to the accompanying diagrammatic drawings, of which;
Figure 1 is a view of a person wearing an orthotic device,
Figure 2 is a side elevation of members of an orthotic device according to the present invention,
Figure 3 is an end elevation (partly in section along line III-III of Figure 2,) corresponding to figure 2,
Figure 4 is a detail of one of the members shown in Figure 3,
Figure 5 is an end view, in section along line V-V corresponding to figure 4,
Figure 6 is an elevation, in section along line Vl-VI of figure 3,
Figure 7 is a view from the bottom of a plunger member illustrated in fugure 6,
Figure 8a is a perspective view of a cross-linking device adapted for use in the invention,
Figure 8b, is a detail, in the direction of arrow B in Figure 8a, of the cross-linking device, and
Figure 9 is a detail, partly in section and corresponding to Figure 3, of the attachment of the cross-linking device to the members of the orthotic device.

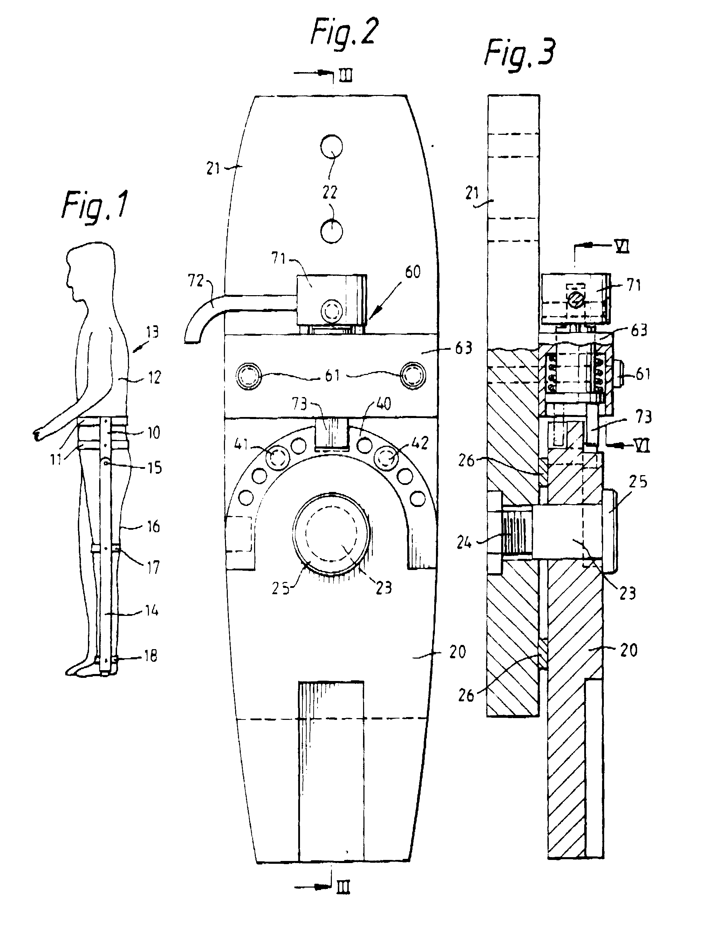

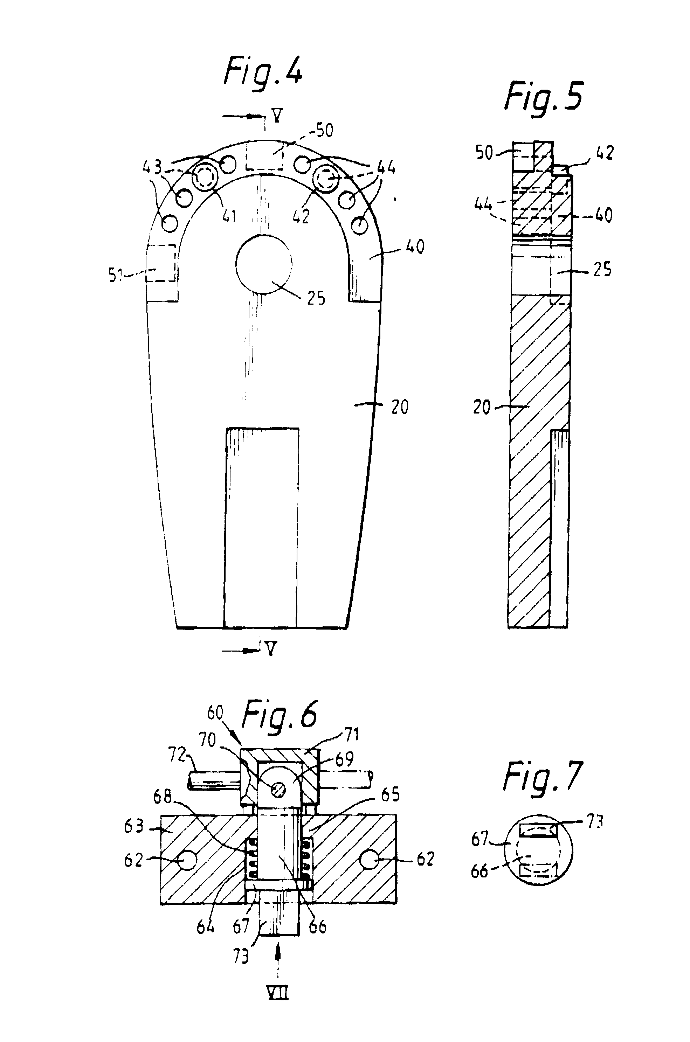

A hip support orthotic device (figure 1) has a first rigid beam 10 attached by a brace 11 to a torso 12 of a patient shown generally at 13. A second beam 14 is rotatably connected to the first beam 10 at a pivot point 15 ajacent a hip joint of the patient 13 and is secured to a leg 16 by straps 17,18. There will normally be a similar device (not shown) also attached to the brace member 11 and supporting the other hip of the patient 13. At the pivot point 15, shown in more detail in figures 2 and 3, a first member 20, secured to the second beam 14 (by means not shown) is rotatably attached to a second member 21 (secured to the first beam member 10 by means of bolts (not shown) passing through holes 22) by means of an axle 23 having a threaded end portion 24. which mates with a threaded section in the second member 21. and a head 25 to maintain the first member 20 in a position rotatable around the axle 23 relative to the second member 21. Friction pads 26 may be positioned between the first 20 and second 21 members. The first member 20 (see particularly figures 4 and 5) has an annular channel 40 extending along an arc of a circle of constant radius relative to the axis of the axle 23. Two stop members 41,42 (figure 2) can be positioned at any one of a plurality of positions 43,44 (figure 4) respectively. The stop members 31,32 might be, for example: screws fitting into tapped bores constituting the positions 43, 44.

On a side of the first member 20 opposite to the annular channel 40 are 2 rectangular slots 50, positioned generally along an axis passing through the pivot point 15 and along the second beam 14 (see particularly figure 5) and 51 (in dotted lines on figure 4) roughly at right angles. relative to the pivot point 15,to the first slot 50.

A control block 60, shown in detail in figures 6 and 7, is secured to the second member 21 (figure 2) by bolts 61 passing through holes 62 in a body 63 (figure 6). A circular bore 64 through the body 63 has at one end a flange 65 in which is slideable a cylindrical plunger 66 having adjacent one end a flange 67 which slides in the bore 64. A spring 68 in compression between the flanges 65, 67 biases the plunger away from the flange 65. At an end of the plunger 66 adjacent the flange 65 is secured a bracket 69 to a pivot 70 of which is attached a control member 71 rotatably mounted on the body 63. A lever 72 is attached to the control member 71, protruding from the other end of the plunger 66 is a tongue 73 positioned along a chord of the plunger at a distance from a centre line of the plunger (see particularly figure 7). The control member 60 is so positioned relative to the first member 20 that , depending on the orientation of the control member 71 the tongue 73 protrudes into the annular channel 40 on the first member 20 or into one of the slots 50,51 on the first member 20.

In use the orthopaedic device is fitted to a patient and the stops 41, 42 in the annular channel 40 on the first member 20 set at an arbitrary position. The control member 71 is adjusted such that the tongue 73 protrudes into the channel 40 between the stops 41, 42. A specialist will then ask the patient to carry out tests, and will have the stops 41, 42 repositioned in the various positions 43, 44 respectively until he considers the positions optimised. The stops 41, 42 can then be left in position until a change in the patient's condition results in a need for reassessment and readjustment. This can again be carried out in situ. The stops 41, 42 can be overriden by operation of the lever 72 such that the control member 71 pivots about an end opposite to the lever 72 raising the piston 66 relative to the body 63 until the tongue 73 moves out of the channel 40. Rotation of the control member 71 through 180°, with the piston in the raised position, moves the tongue 73 out of alignment with the channel 40, where it contacts the stop members 41, 42, into alignment with the slots 50,51. When the patient is in the standing position plunger 66 and hence tongue 73 can then be lowered so that the tongue 73 locks into the slot 50 so locking the device in a position corresponding to the patient's standing. Likewise the tongue 73 can be positioned in the slot 51 to lock the device into a position corresponding to the sitting position of the patient.

The construction of the first 20 and second 21 members, and associated items, is preferably such that a cross-linking device, such as is common in the art, can be fitted with the device still attached to the patient.

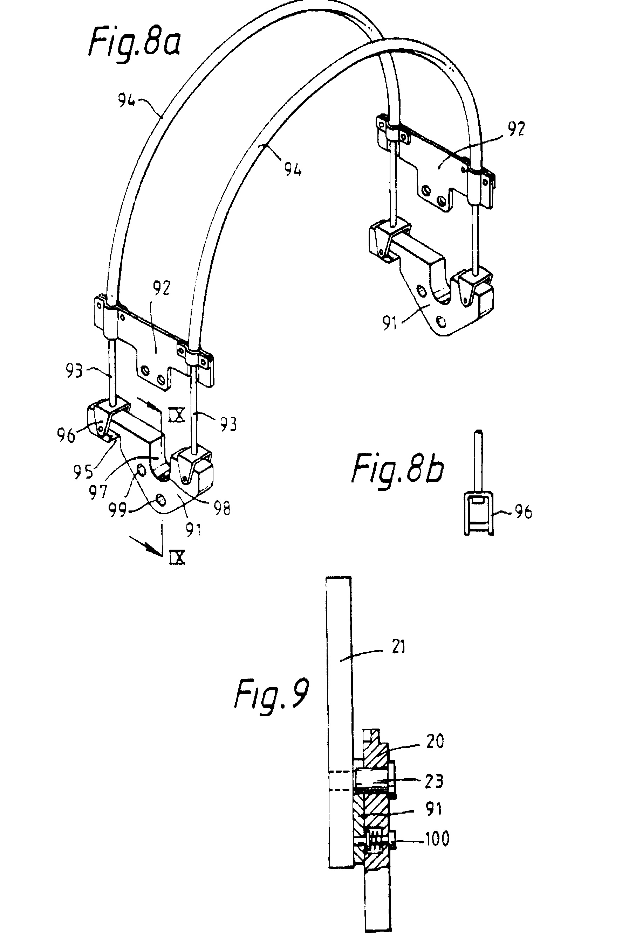

A cross-linking device, adapted for use with the invention (Figures 8a, 8b) includes, for each hip of a patient, a cable attachment member 91 and a sheath attachment member 92. Two sheathes 94 of bowden cables 93 are secured to opposite ends of the sheath attachment members 92 and their enclosed cables 94 secured to the cable attachment members 91. The bowden cables 93 are arranged so that rotation of one cable attachment member 91 in one direction relative to its associated sheath attachment member 92 can only take place when accompanied by rotation of the other cable attachment member 91 in the other direction relative to its associated sheath attachment member 92.

To each cable attachment member 91 one cable 93 is permanently attached, and one releasably attached by means of a hook 95 on the member 91 and a bracket 96 (see Figure 8b) at the end of the cable 93.

In each cable attachment member 91 there is a slot 97 having an end 98 contoured to fit the axle 23, and two holes 99, whose purpose will be described later.

A modified first member 20 (see Figure 9) includes a spring-loaded plunger 100. which may conveniently be of similar design to the piston 66.

The cross-linking device is added to the orthotic device by removing the control blocks 60 and replacing them with the sheath attachment members 92. The cable attachment members 91 are then (with brackets 96 and hooks 95 disconnected, and with, if need be, friction pads 26 removed) positioned between first 20 and second 21 members with the end 98 fitting against the axle 23. The plungers 100 are inserted into holes 99, the holes being selected to correspond to either a sitting or a standing position of a patient wearing the orthotic device. Finally the brackets 96 and hooks 95 are connected.

It will be readily apparent that many modifications can be made to the above described embodiments of the invention whilst remaining with the scope of the invention. For example whilst the stop members 41, 42 have been described as fitting into a plurality of tapped positions 43, 44 arrangements whereby a stop member can be continuously adjusted will be readily apparent to those skilled in the art. For example a nut having a shank passing through an annular slot in the structure of the first member 20 to a nut constrained against rotation might be moved by slackening, moving in the slot and then retightening.

Quick release means whereby the control member 60 can be removed and replaced will be readily apparent to those skilled in the engineering arts.

It may in some treatments be desirable to have the control block 60 left in position when the cross-linking device is attached: means for accomplishing this will be readily apparent (by, for example. having slots in the sheath attachment members 92, which slots fit round the control blocks 60, and means similar to the control block 60 securement means 61, 62 for securing the sheath attachment members 92 to the second members 21)

The orthotic device, with or without the cross-linking device attached may, for treatment of some patients, advantageously have means for attaching various spring and damper devices These might include, for example, an interconnected two-way damper operating on both hip joints. a pair of independant dampers operating on individual hip joints, a single spring or gas strut acting on an individual hip joint, a pair of independant springs or gas struts acting individually on hip joints, and various combinations of coupled spring and damper units.

## Claims

1. An orthotic device, for the investigation and treatment of joint malfunctions, including a first member rotatably connected to a second member, means for allowing relative rotation of the first and second members over a range between two adjustable limits and over-ride means for allowing relative rotation of the first and second members beyond these limits, and locking means for locking the first and second members in at least one fixed position.

2. An orthotic device as claimed in Claim 1 wherein the adjustable limits can be adjusted whilst the device is fitted to a patient.

3. An orthotic device as claimed in Claim 1 or in Claim 2 wherein the over-ride means and the locking means can be operated by a wearer of the device.

4. An orthotic device is claimed in any one of Claims 1 to 3 wherein the adjustable limits are determined by two stop screws whose position in one of the first or second members can be varied along an arc whose centre is the centre of relative rotation of the first and second members.

5. An orthotic device as claimed in Claim 4 wherein the other of the first and second members includes plunger means which can be entended into the arc between the two stop screws and which can be retracted to override the limits.

6. An orthotic device as claimed in Claim 5 wherein the plunger is offset with respect to an axis about which it can be rotated out of alignment with the arc and into alignment with at least one slot into which it can be positioned to lock the first and second members.

7. A walking aid including two orthotic devices, each as claimed in any one of Claims 1 to 6, one for each hip of a patient.

8. A walking aid as claimed in Claim 7 wherein the orthotic devices can, whilst the aid is being worn by a patient, be adapted to h¿:¡e fitted, and can have fitted thereto a cross-linking device.

9. A walking aid as claimed in Claim 8 wherein the cross-linking device is of the type including two crossed bowden cables.

10. A walking aid as claimed in Claim 9 wherein the cross-linking device is fitted to the walking aid by securing a bowden sheath attachment member to one of the first or second members of each orthotic device, by positioning a bowden cable attachment member between the first or second members and by constraining the bowden cable attachment device to move with the other of the first and second members.

11. A walking aid as claimed in Claim 10 wherein the bowden cable attachment device can be constrained to move with the other of the first and second members in a position corresponding to a walking attitude of the patient.

12. A walking aid as claimed in Claim 10 or in Claim 11 wherein, the bowden cable attachment device can be constrained to move with the other of the first and second members in a position corresponding to a sitting attitude of the patient.

13. A walking aid as claimed in any one of Claims 7 to 12 including at least one of a spring, a damper and a gas strut.

14. An orthotic device substantially as herein described with reference to Figures 2 to 7 of the accompanying drawings.

15. An orthotic device substantially as herein described.

16. A walking aid substantially as herein described with reference to Figures 1 to 9 of the accompanying drawings.

17. A walking aid substantially as herein described.
